(19) Europäisches Patentamt
European Patent Office
Office européen des brevets

(11) **EP 4 439 046 A1**

(12) **EUROPEAN PATENT APPLICATION**

(43) Date of publication:
**02.10.2024 Bulletin 2024/40**

(21) Application number: **23165395.7**

(22) Date of filing: **30.03.2023**

(51) International Patent Classification (IPC):
*G01N 21/31* (2006.01)   *G01N 33/04* (2006.01)

(52) Cooperative Patent Classification (CPC):
**G01N 21/31; A61B 5/0075; A61B 5/0082;
A61B 5/14507; A61B 5/14546; A61B 5/1455;
A61B 5/4288; A61M 1/06; G01J 3/02;
G01N 33/487; A61M 2202/08; A61M 2205/3313;
A61M 2205/52; G01N 21/3151**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **FERNANDO, Shakith Devinda**
**Eindhoven (NL)**

• **VAN BREE, Karl Catharina**
**Eindhoven (NL)**
• **LUCASSEN, Gerhardus Wilhelmus**
**Eindhoven (NL)**
• **PAULUSSEN, Elvira Johanna Maria**
**Eindhoven (NL)**

(74) Representative: **Philips Intellectual Property &
Standards
High Tech Campus 52
5656 AG Eindhoven (NL)**

(54) **SYSTEM AND METHOD FOR DETERMINING CHARACTERISTICS OF A MILK-CONTAINING SYSTEM**

(57) A system and method for determining one or more characteristics of a milk-containing system. A distribution of photon arrival times for at least two wavelengths is obtained from at least one SPAD sensor configured to detect photons arriving from within the milk-containing system. The at least two wavelengths correspond to absorption of two of water, lipids and beta carotene. The distribution of photon arrival times is processed to determine one or more characteristics of the milk-containing system.

FIG. 1

**Description**

FIELD OF THE INVENTION

The invention relates to the field of determining characteristics of a milk-containing system, and in particular to characteristics of a system containing human breast milk.

BACKGROUND OF THE INVENTION

Human breast milk contains all the most important nutrients for human newborns and infants. However, only 40% of mothers exclusively breastfeed during the first six months from birth (see WHO and UNICEF, "Global Breastfeeding Scorecard, 2019: Increasing commitment to breastfeeding through funding and improved policies and programmes", WHO/NMH/NHD/19.22).

There are a number of reasons for the low rate of exclusive breastfeeding. Many of these reasons relate to experiencing difficulties in breastfeeding. In particular, many mothers have a perception that their milk supply is insufficient. Some mothers experience lactiferous duct blockage. Mothers who use breast pumps can find that the breast pump is not suitable for their milk ejection reflex.

There is therefore a need for improved aid for breastfeeding mothers.

SUMMARY OF THE INVENTION

The invention is defined by the claims.

According to examples in accordance with an aspect of the invention, there is provided a processing system for determining one or more characteristics of a milk-containing system, the processing system being configured to: obtain, from at least one SPAD sensor, a distribution of photon arrival times at a plurality of pixels of the at least one SPAD sensor for photons emitted by a light source and reflected from within or transmitted through the milk-containing system; and process the distribution of photon arrival times to determine one or more characteristics of the milk-containing system, wherein the photons comprise first photons having a wavelength within a first wavelength range and second photons having a wavelength within a second, different wavelength range, wherein each of the first wavelength range and the second wavelength range is a different one of the following: a wavelength range coinciding with an absorption peak of water; a wavelength range coinciding with an absorption peak of lipids; and a wavelength range coinciding with an absorption peak of beta carotene.

The inventors have recognized that photon counting with time-domain spectroscopy can be used to provide information relating to a milk-containing system. In particular, the milk-containing system may contain human breast milk. For instance, the milk-containing system may be a lactating breast or a bottle of expressed breast milk.

This information may, for example, be provided to a breastfeeding mother, allowing the mother to make informed decisions regarding their breastfeeding, or used to generate breastfeeding guidance for the mother for an enhanced breastfeeding experience.

The one or more characteristics may, for example, include a lipid content of the milk, a beta carotene content of the milk, a water content of the milk and/or a riboflavin content of the milk. In the case where the milk-containing system comprises a lactating breast, the one or more characteristics may additionally or alternatively include one or more of a time of a milk ejection reflex event, a milk duct flow, a location of a milk duct blockage, a severity of a milk duct blockage and/or a change in milk supply over time.

In some examples, the milk-containing system is a breast of a human subject; and the processing system is configured to determine the one or more characteristics by: processing the distribution of photon arrival times to generate a time-varying milk duct dilation signal; and processing the time-varying milk duct dilation signal to determine the one or more characteristics.

The inventors have recognized that milk duct dilation during milk ejection is correlated with milk flow, and that a milk duct dilation signal can be generated using photon counting with time-domain spectroscopy.

This technique has numerous advantages over existing techniques for measuring milk duct dilation. In particular, the use of light provides a safer measurement than ultrasound, and a light source and SPAD sensor for obtaining the data relating to the distribution of photon arrival times may be provided at a lower cost than an ultrasound system. Compared with existing optical techniques (e.g. CMOS image sensing or OCT-Doppler flow imaging), photon counting using a SPAD sensor allows fast changes in milk duct diameter during milk ejection reflex events to be detected and provides three-dimensional depth information.

In some examples, the photons further comprise third photons having a wavelength within a third wavelength range, different to the first and second wavelength ranges, wherein each of the first wavelength range, the second wavelength range and the third wavelength range is a different one of the following: a wavelength range coinciding with an absorption peak of water; a wavelength range coinciding with an absorption peak of lipids; and a wavelength range coinciding with an absorption peak of beta carotene; and the processing system is configured to generate the milk duct generation signal by: for an initial time window: processing the distribution of photon arrival times to determine, for each pixel, a first absorption coefficient for each of the first, second and third wavelength ranges; processing the first absorption coefficients to determine, for each pixel, a concentration value for each of water, lipids and beta carotene; processing the concentration values for each pixel to identify pixels corresponding to a milk duct; and, for subsequent time windows: processing the data relating to the distribution of photon arrival times at each time window to determine a second absorption coefficient for one of the first, second and third wavelength ranges for at least one pixel of the identified pixels; processing each second absorption coefficient for each time window for the at least one pixel to generate the milk duct dilation signal.

This embodiment is based on the recognition that the concentration of milk is correlated with milk duct dilation. Preferably, a distribution of photon arrival times from a SPAD sensor positioned at a front of the nipple is used for this embodiment.

The inventors have further recognized that the concentration values can be used to distinguish between breast milk in a milk duct and breast tissue, since the concentrations of water, lipids and beta carotene are significantly different for the two. The concentration of milk is then generated based on concentration values for the milk duct.

In some examples, the photons further comprise fourth photons having a wavelength within a fourth wavelength range coinciding with an absorption peak of oxyhemoglobin and fifth photons having a wavelength within a fifth wavelength range coinciding with an absorption peak of deoxyhemoglobin; the second absorption coefficient is determined for the wavelength range coinciding with an absorption peak of lipids; and the processing system is further configured to: process the distribution of photon arrival times to determine an oxyhemoglobin and deoxyhemoglobin absorption coefficient; and generate the milk duct dilation signal by processing the second absorption coefficient and the oxyhemoglobin and deoxyhemoglobin absorption coefficient.

Since oxyhemoglobin and deoxyhemoglobin have a similar absorption coefficient to lipids, the accuracy of a milk duct dilation signal generated based on a lipids absorption coefficient can be improved by normalizing the lipids absorption coefficient using the oxyhemoglobin and deoxyhemoglobin absorption coefficient.

In some examples, the processing system is configured to generate the milk duct dilation signal by: processing the distribution of photon arrival times to determine a change in the distribution over time for each of the first and second wavelength ranges; and processing the change in the distribution over time for each of the first and second wavelength ranges to generate the milk duct dilation signal.

This embodiment is based on the recognition that the depth of the milk duct varies with dilation. Since the distribution of arrival times is correlated with tissue depth, changes in the distribution are correlated with changes in the diameter of the milk duct as the milk duct dilates. Preferably, a distribution of photon arrival times from a SPAD sensor positioned at a side of the nipple is used for this embodiment.

The change in the distribution over time for each of the first and second wavelength ranges may comprise a change in a peak of the distribution or a variation in a time-spread of the distribution.

In some examples, the processing system is further configured to filter the distribution of photon arrival times based on predetermined anatomical information.

The use of predetermined anatomical information allows the distribution to be filtered to remove at least some of the photons reflected from breast tissue.

The predetermined anatomical information may, for instance, include a typical anatomical depth of lipids and/or a typical anatomical depth of a milk duct.

In some examples, the processing system is configured to process the time-varying milk duct dilation signal by: receiving at least one historical time-varying milk duct dilation signal for the subject; and processing the time-varying milk duct dilation signal and the historical time-varying milk duct signal to determine the one or more characteristics.

This allows long-term changes in milk supply to be determined.

In some examples, the processing system is configured to: generate a plurality of milk duct dilation signals, each corresponding to a different subset of pixels; and process the plurality of milk duct dilation signals to determine the one or more characteristics.

In some examples, the at least one SPAD sensor comprises a plurality of SPAD sensors, each configured to obtain a distribution of photon arrival times for photons reflected from a different region of the breast; and the processing system is configured to: generate a plurality of milk duct dilation signals, each generated using the distribution of photon arrival times of a different SPAD sensor; and spatially correlate the plurality of milk duct dilation signals to identify a location of a milk duct blockage.

Early identification of milk duct blockages can reduce a likelihood of mastitis occurring.

In some examples, the one or more characteristics comprise a lipids content, a water content and/or a beta carotene content of milk in the milk-containing system; the photons further comprise third photons having a wavelength within a third wavelength range, different to the first and second wavelength ranges, wherein each of the first wavelength range, the second wavelength range and the third wavelength range is a different one of the following: a wavelength range coinciding with an absorption peak of water; a wavelength range coinciding with an absorption peak of lipids; and a wavelength range coinciding with an absorption peak of beta carotene; and the processing system is configured to determine the lipids content, water content and/or beta carotene content of milk in the milk-containing system by: processing the distribution of photon arrival times to determine an absorption coefficient for each of the first, second and third wavelength ranges; processing the absorption coefficients to determine a concentration value for each of water, lipids and beta carotene; and processing the concentration values to determine a lipids content, a water content and/or a beta carotene content of milk in the milk-containing system.

The lipids content and/or beta carotene content of milk can provide important information regarding the nutritional content of a mother's breast milk. The lipids content and/or beta carotene content may be determined from in vivo or in vitro embodiments.

In some examples, the processing system is further configured to process the one or more characteristics to generate breastfeeding guidance.

Where the milk-containing system comprises a lactating breast, the guidance may, for instance, relate to milk supply issues and/or milk duct blockages.

Breastfeeding guidance may also be provided based on the characteristics of expressed human breast milk. For instance, a recommendation to adjust a mother's diet may be generated based on the lipid or beta carotene content of the expressed milk.

There is also proposed a system for determining one or more characteristics of a milk-containing system, the system comprising: a light source configured to emit photons towards the milk-containing system, wherein the light source is configured to emit photons comprising first photons having a wavelength within a first wavelength range and second photons having a wavelength within a second, different wavelength range, wherein each of the first wavelength range and the second wavelength range is a different one of the following: a wavelength range coinciding with an absorption peak of water; a wavelength range coinciding with an absorption peak of lipids; and a wavelength range coinciding with an absorption peak of beta carotene; at least one SPAD sensor, configured to measure an arrival time of photons reflected from within the milk-containing system; and the processing system described above.

There is also proposed a nipple-interface device, comprising: a cavity configured to receive a nipple; a light source configured to emit photons towards the cavity, wherein the light source is configured to emit photons comprising first photons having a wavelength within a first wavelength range and second photons having a wavelength within a second, different wavelength range, wherein each of the first wavelength range and the second wavelength range is a different one of the following: a wavelength range coinciding with an absorption peak of water; a wavelength range coinciding with an absorption peak of lipids; and a wavelength range coinciding with an absorption peak of beta carotene; and at least one SPAD sensor, configured to measure an arrival time of reflected photons.

A nipple-interface device may, for instance, be a breast pump (for determining the one or more characteristics during breast pumping), a nipple shield (for determining the one or more characteristics during breastfeeding), a lactation massage device or a breast milk collector.

According to another aspect of the invention, there is provided a method for determining one or more characteristics of a milk-containing system, the method comprising: obtaining, from at least one SPAD sensor, a distribution of photon arrival times at a plurality of pixels of the at least one SPAD sensor for photons emitted by a light source and reflected from within the milk-containing system, wherein the photons comprise first photons having a wavelength within a first wavelength range and second photons having a wavelength within a second, different wavelength range, wherein each of the first wavelength range and the second wavelength range is a different one of the following: a wavelength range coinciding with an absorption peak of water; a wavelength range coinciding with an absorption peak of lipids; and a wavelength range coinciding with an absorption peak of beta carotene; and processing the distribution of photon arrival times to determine one or more characteristics of the milk-containing system.

There is also proposed a computer program product comprising computer program code means which, when executed on a computing device having a processing system, cause the processing system to perform all of the steps of the method described above.

These and all other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 illustrates a system for determining one or more characteristics of a milk-containing system, according to an embodiment of the invention;

Fig. 2 illustrates a first method for generating a time-varying milk duct dilation signal;

Fig. 3 illustrates an example decision tree for classifying pixels as corresponding to a milk duct or breast tissue;

Fig. 4 illustrates a second method for generating a time-varying milk duct dilation signal;

Fig. 5 illustrates a computer-implemented method for determining one or more characteristics of a milk-containing system, according to an embodiment of the invention;

Fig. 6 illustrates a system for determining one or more characteristics of a milk-containing system, according to another embodiment of the invention;

Fig. 7 illustrates a system for determining one or more characteristics of a milk-containing system, according to another embodiment of the invention; and

Fig. 8 illustrates a system for determining one or more characteristics of a milk-containing system, according to another embodiment of the invention.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

There is proposed a system and method for determining one or more characteristics of a milk-containing system. A distribution of photon arrival times for at least two wavelengths is obtained from at least one SPAD sensor configured to detect photons arriving from within the milk-containing system. The at least two wavelengths correspond to absorption of two of water, lipids and beta carotene. The distribution of photon arrival times is processed to determine one or more characteristics of the milk-containing system.

Embodiments are at least partly based on the realization that photon counting with time-domain near-infrared spectroscopy can provide information about the composition of milk within a milk-containing system, and information about milk ducts within a human breast (e.g. milk ejection reflex events and milk flow).

Illustrative embodiments may, for example, be employed in breastfeeding systems and in breast milk expression systems.

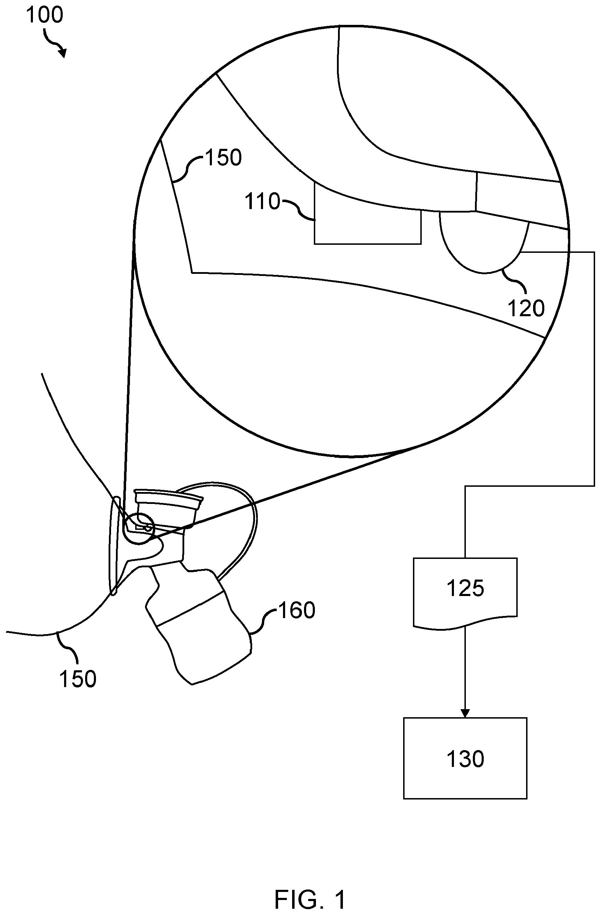

Fig. 1 illustrates a system 100 for determining one or more characteristics of a milk-containing system 150, according to an embodiment of the invention. The system 100 comprises a light source 110, at least one SPAD sensor 120, and a processing system 130. The processing system is, itself, an embodiment of the invention.

In Fig. 1, the milk-containing system 150 is a breast of a human subject. However, the system 100 is not limited to the determination of characteristics of a lactating breast, and the milk-containing system may be any system that contains milk, in particular human breast milk. For example, the milk-containing system may comprise a bottle of expressed breast milk.

The light source 110 is configured to emit photons towards the milk-containing system. The photons emitted by the light source comprise photons of at least two different wavelengths: a first wavelength within a first wavelength range, and a second, different wavelength within a second wavelength range. The first and second wavelength ranges are each a different one of: a wavelength range coinciding with an absorption peak of water; a wavelength range coinciding with an absorption peak of lipids; and a wavelength range coinciding with an absorption peak of beta carotene.

Suitable values for these wavelength ranges will be apparent to the skilled person. For instance, a wavelength range of 950-1000 nm may be used as a wavelength range coinciding with an absorption peak of water; a wavelength range of 900-950 nm may be used as the wavelength range coinciding with an absorption peak of lipids; and a wavelength range of 450-550 nm may be used as the wavelength range coinciding with an absorption peak of beta carotene.

When the milk-containing system 150 is a human breast, wavelengths in the ranges of 900-950 nm and 950-1000 nm will penetrate sufficiently deeply to provide information on most milk ducts (which have a depth distribution of around 1-8 mm). A wavelength in the range of 450-550 nm has a smaller penetration depth, so will only provide information on superficial milk ducts; however, this information may be sufficient for determining the one or more characteristics of the milk-containing system, especially given that the superficial milk ducts dilate and expand during milk ejection.

Suitable light sources for use as the light source 110 will be apparent to the skilled person. For instance, the light source may comprise a plurality of lasers, each laser configured to emit photons of a different wavelength.

The photons emitted by the light source are reflected (e.g. backscattered) from within or transmitted through the milk-containing system 150. The at least one SPAD sensor 120 is configured to measure an arrival time of these reflected/transmitted photons.

A SPAD (Single Photon Avalanche Diode) sensor is a type of image sensor that detects each individual photon incident on a pixel of the sensor. Each photon that enters a pixel is converted to an electric charge, and the resulting electrons are multiplied to form a large signal charge. The use of a SPAD sensor allows the time-of-flight of individual photons to be recorded; for instance, SPAD sensing has been used in brain imaging, with the direct time-of-flight computation using blood oxygenation as a marker (see Ban et al. (2022), "Kernel Flow: a high channel count scalable time-domain functional near-infrared spectroscopy system", J Biomed Opt, 27(7):074710).

In Fig. 1, the light source 110 and the at least one SPAD sensor 120 are integrated within a breast pump 160. However, the invention is not limited to this particular example, and the light source and at least one SPAD sensor may each be located in any suitable device.

For instance, where the milk-containing system is a human breast, the light source and at least one SPAD sensor may be contained within any suitable nipple-interface device comprising a cavity configured to receive a nipple. The light source may be configured to emit photons towards the cavity (i.e. towards a nipple received within the cavity). Examples of suitable nipple-interface devices include breast pumps, nipple shields, lactation massage devices and breast milk collectors (e.g. let-down collectors). A nipple interface device comprising a light source and at least one SPAD sensor as described herein is, itself, an embodiment of the invention.

The light source and at least one SPAD sensor may alternatively be contained in a dedicated milk-analysis device, or in separate devices. This may, in particular, be the case when the milk-containing system is outside the human body (e.g. where the milk-containing system is a bottle of expressed breast milk).

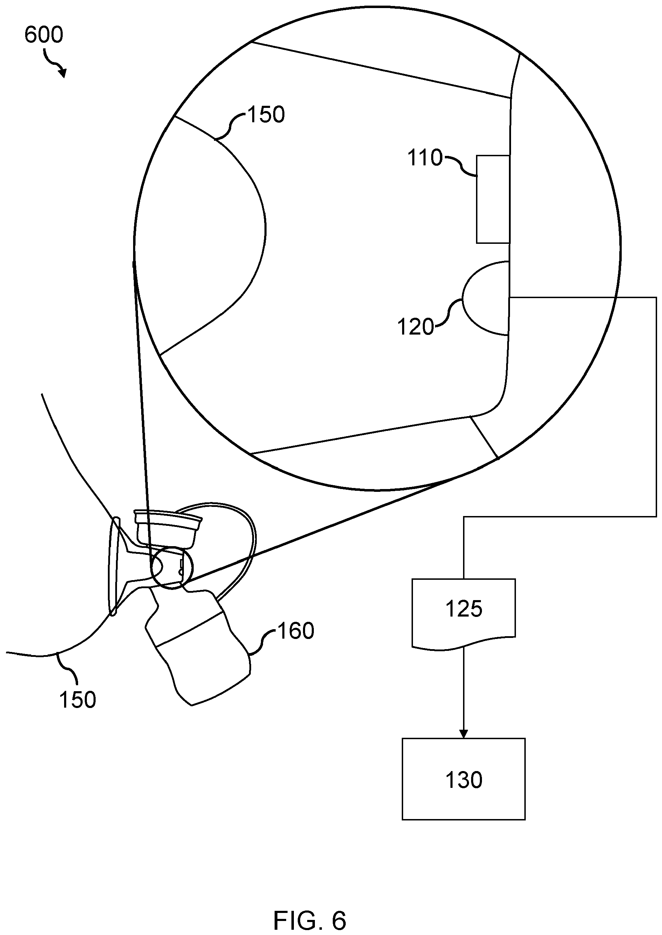

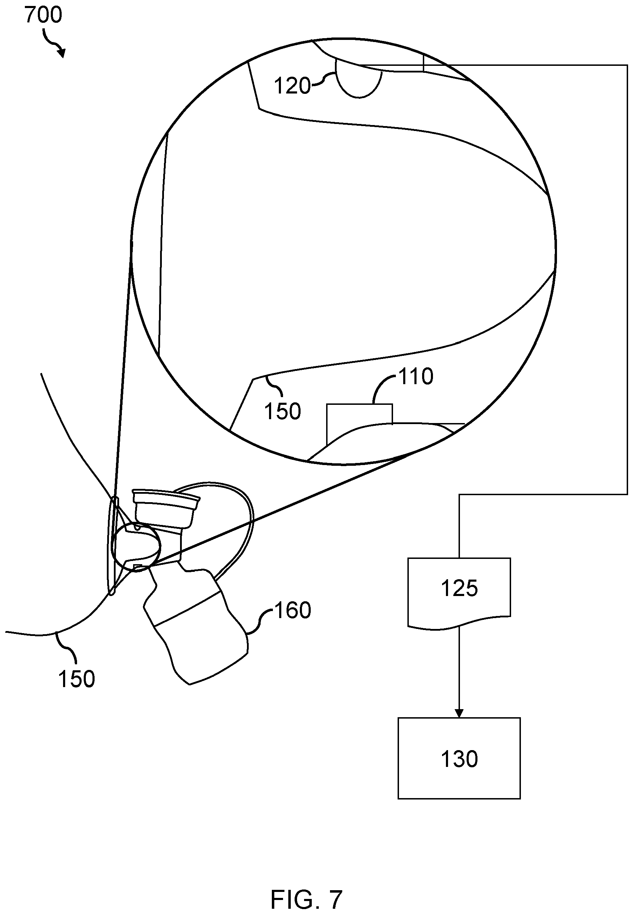

In Fig. 1, the light source 110 and the at least one SPAD sensor 120 are positioned at a side of the nipple (i.e. such that the direction of travel of the photons is substantially perpendicular to a direction of milk flow). The light source and the at least one SPAD sensor are positioned at a same side of the nipple, such that the at least one SPAD sensor detects photons emitted by the light source and reflected from within the milk-containing system. In other examples, the light source 110 and the at least one SPAD sensor 120 may be positioned at a front of the nipple (i.e. such that the direction of travel of the photons is substantially parallel to a direction of milk flow)(see Fig. 6), or the and light source and SPAD sensor can be arranged in an rectilinear set-up, where light source is on one side of the nipple area and the SPAD sensor on an opposite side (see Fig. 7).

Preferably, the light source 110 is configured to emit photons from a fixed position with respect to the milk-containing system. For instance, where the light source and the at least one SPAD sensor are provided in a breast pump, the emission of photons from the light source may be synchronized to the pumping frequency of the breast pump.

The processing system 130 is configured to obtain, from the at least one SPAD sensor 120, a distribution 125 of photon arrival times at a plurality of pixels of the at least one SPAD sensor for photons emitted by the light source and reflected or scattered from within the milk-containing system.

Having received the distribution 125 of photon arrival times, the processing system 130 is configured to process the distribution of photon arrival times to determine one or more characteristics of the milk containing system 150.

The one or more characteristics of the milk-containing system may comprise any characteristics that can be determined based on the distribution of photon arrival times from the milk-containing system 150. For instance, the one or more characteristics may comprise nutritional information relating to the milk within the milk-containing system, such as a lipid content, a beta carotene content, a water content and/or a riboflavin content (e.g. a percentage content). If the milk-containing system is a human breast, the one or more characteristics may additionally or alternatively comprise one or more breastfeeding characteristics, such as a time of a milk ejection reflex event, a milk duct flow, a location of a milk duct blockage, a severity of a milk duct blockage, and/or a change in milk supply over time.

In some examples, the one or more characteristics may comprise nutritional information relating to the composition of milk within the milk-containing system 150. These characteristics may be determined for any type of milk-containing system (i.e. for both in vivo and in vitro systems).

In order to determine this nutritional information, at least three different wavelengths are required, the wavelengths corresponding to water, lipids and beta carotene respectively. In other words, the photons emitted by the light source 110 further comprise photons having a wavelength within a third wavelength range, different to the first and second wavelength ranges, such that the photons emitted by the light source comprise photons having a wavelength within a wavelength range coinciding with an absorption peak of water, photons having a wavelength within a wavelength range coinciding with an absorption peak of lipids, and photons having a wavelength within a wavelength range coinciding with an absorption peak of beta carotene.

The processing system 130 is configured to determine a lipids content, a beta carotene content, and/or a water content of milk in the milk-containing system by processing the distribution 125 of photon arrival times to determine an absorption coefficient for each of the first, second and third wavelength ranges (i.e. absorption coefficients for water, lipids and beta carotene), and processing the absorption coefficients to determine a concentration value for each of water, lipids and beta carotene.

The absorption coefficients for water, lipids and beta carotene may, for instance, be determined by processing the distribution 125 of photon arrival times to determine the optical properties of the substance which reflected or scattered the photons using the method described in Liebert et al. (2003), "Evaluation of optical properties of highly scattering media by moments of distributions of times of flight of photons", Applied Optics, 42(28):5785-5792.

The concentration values for water, lipids and beta carotene may be determined from the absorption coefficients by applying a modification of the Beer-Lambert law, as expressed by the equation:

$$c = \frac{\mu_{10}(\lambda)}{\varepsilon(\lambda)} \tag{1}$$

where c is the concentration, $\mu_{10}(\lambda)$ is the absorption coefficient at wavelength $\lambda$, and $\varepsilon(\lambda)$ is the expected molar absorption coefficient. The skilled person will be readily capable of determining an expected molar absorption coefficient for each of water, lipids and beta carotene.

The concentration values for water, lipids and beta carotene are processed for each wavelength to determine a water content, a lipids content and/or a beta carotene content.

In some examples, the distribution 125 of photon arrival times further comprises photons having at least one additional wavelength within a wavelength range coinciding with an absorption peak of riboflavin (e.g. a wavelength range of 500-800 nm), and the processing system 130 is further configured to determine a riboflavin content of milk in the milk-containing system. The processing system may determine the riboflavin content using the method described above with respect to water, lipids and beta carotene.

In some examples in which the milk-containing system is a human breast, the one or more characteristics may additionally or alternatively comprise one or more breastfeeding characteristics, such as a time of a milk ejection reflex event, a milk duct flow, a location of a milk duct blockage, a severity of a milk duct blockage, and/or a change in milk supply over time. The processing system 130 may be configured to determine these breastfeeding characteristics by processing the distribution 125 of photon arrival times to generate a time-varying milk duct dilation signal, and processing the time-varying milk duct dilation signal to determine the one or more characteristics.

A time-varying milk duct dilation signal is a signal responsive to changes in milk duct diameter. Various methods are proposed for generating a time-varying milk duct dilation signal based on the distribution 125 of photon arrival times.

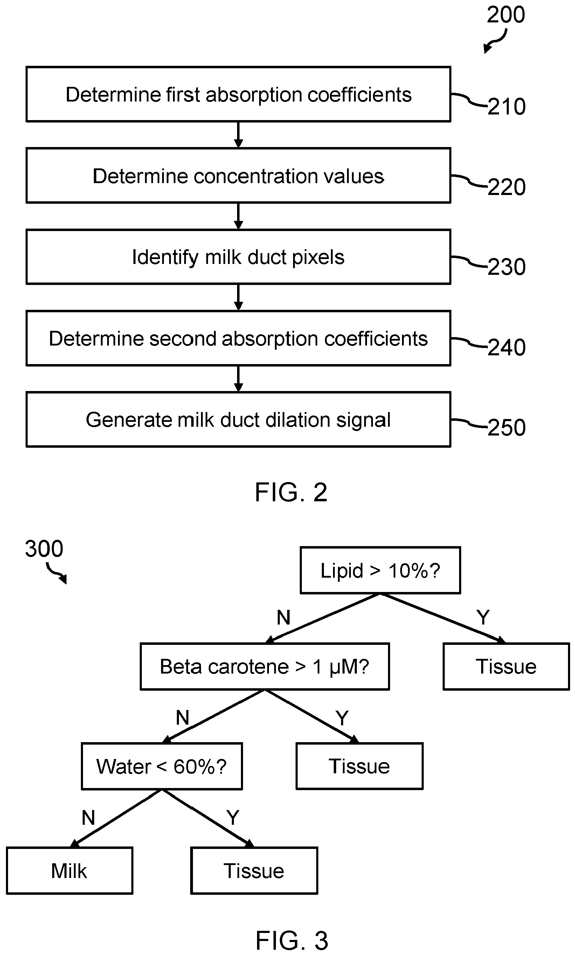

Fig. 2 illustrates a first method 200 for generating a time-varying milk duct dilation signal. This first method for generating a time-varying milk duct dilation is preferred when the SPAD sensor is positioned in front of the nipple.

At least three different wavelengths (corresponding to water, lipids and beta carotene respectively) are required to generate a time-varying milk duct dilation signal using this first method. In other words, the distribution 125 of photon arrival times used in this first method comprises arrival times for photons having a wavelength within a wavelength range coinciding with an absorption peak of water, photons having a wavelength within a wavelength range coinciding with an absorption peak of lipids, and photons having a wavelength within a wavelength range coinciding with an absorption peak of beta carotene.

The method 200 begins at step 210, in which the distribution of photon arrival times is processed, for an initial time window (e.g. a first sampling window), to determine, for each pixel of the at least one SPAD sensor, a first absorption coefficient for each of the three wavelength ranges.

In other words, an absorption coefficient for water is determined based on the arrival times of photons having a wavelength within the wavelength range coinciding with an absorption peak of water; an absorption coefficient for lipids is determined based on the arrival times of photons having a wavelength within the wavelength range coinciding with an absorption peak of lipids; and an absorption coefficient for beta carotene is determined based on the arrival times of photons having a wavelength within the wavelength range coinciding with an absorption peak of beta carotene.

At step 220, the first absorption coefficients are processed to determine, for each pixel of the SPAD sensor, a concentration value for each of water, lipids and beta carotene, for example, using the method described above.

At step 230, the concentration values for each pixel are processed to identify pixels corresponding to a milk duct (i.e. pixels at which the photons arrived from within the milk duct). The concentration values for water, lipids and beta carotene for each pixel may be compared with threshold or reference values to identify pixels corresponding to a milk duct.

The inventors have recognized that the concentration values for each pixel can be used to distinguish between milk (in milk ducts) and breast tissue, since milk and breast tissue have different relative amounts of water, lipids and beta carotene.

Known concentration values for lipids, water and beta carotene in breast tissue and milk ducts may be used to determine suitable threshold or reference values for determining whether each pixel corresponds to a milk duct or breast tissue (see, for example Nachabé et al. (2011), "Diagnosis of breast cancer using diffuse optical spectroscopy from 500 to 1600 nm: comparison of classification methods", J Biomed Opt 16(8):087010; Bosschaart et al. (2019), "Diffuse optical spectroscopic imaging for the investigation of human lactation physiology: a case study on mammary involution", J Biomed Opt 24(5): 1-8; and Xavier et al. (2019), "In Vitro Digestion of Human Milk: Influence of the Lactation Stage on the Micellar Carotenoids Content", Antioxidants 8(8):291).

Fig. 3 illustrates an example decision tree 300 for classifying pixels as corresponding to a milk duct or breast tissue. Using decision tree 300, a lipids content of more than 10%, a beta carotene content of more than 1 $\mu$M, and/or a water content of less than 60% indicates breast tissue. Pixels for which the lipids content is less than 10%, the beta carotene content is less than 1 $\mu$M, and the water content is more than 60% are identified as pixels corresponding to milk ducts. The lipids content and water content may be determined as a percentage from the concentration values as described above.

As the skilled person will appreciate, the decision tree 300 of Fig. 3 illustrates only one set of suitable values for distinguishing between milk and breast tissue, and other, similar values may be used. For instance, suitable values for a threshold to distinguish between breast tissue and milk ducts may be in the range 10-80% for lipids, 10-12% for beta carotene and 30-60% for water.

Returning to Fig. 2, at step 240, the distribution 125 of photon arrival times is processed, at each subsequent time window, to determine a second absorption coefficient for at least one of water, lipids and/or beta carotene for at least one pixel of the identified milk duct pixels. The second absorption coefficient may be determined using the same technique used to determine the first absorption coefficients.

At step 250, each second absorption coefficient for each time window is processed for the at least one pixel to generate the time-varying milk duct dilation signal. The time-varying milk duct dilation signal is, in this case, a signal responsive to changes in a concentration of water, lipids and/or beta carotene in the at least one milk duct pixel. The concentration of water, lipids and/or beta carotene may be determined from the second absorption coefficient using the method described above.

In other words, having identified the pixels that correspond to milk ducts, the concentration of any one of water, lipids and beta carotene within the milk ducts is sufficient to determine a relative amount of milk within the milk ducts, allowing a signal representative of changes in the amount of milk (i.e. the milk duct dilation signal) to be generated.

As previously mentioned, the second absorption coefficient may be determined for any one of lipids, water and beta carotene. If the second absorption coefficient is an absorption coefficient for lipids (i.e. the second absorption coefficient is determined for the wavelength within the wavelength range coinciding with an absorption peak of lipids), additional wavelengths may be used to remove the time-varying effects of oxyhemoglobin and deoxyhemoglobin from the time-varying milk duct signal.

For instance, the photons emitted by the light source 110 may further comprise photons having a wavelength within a fourth wavelength range coinciding with an absorption peak of oxyhemoglobin and photons having a wavelength within a fifth wavelength range coinciding with an absorption peak of deoxyhemoglobin. The processing system 130 may process the distribution 125 of photon arrival times to determine an absorption coefficient for each of oxyhemoglobin and deoxyhemoglobin for the at least one milk duct pixel, and generate the time-varying milk duct dilation signal by processing the absorption coefficients for lipids, oxyhemoglobin and deoxyhemoglobin for the at least one milk duct pixel to normalize the concentration value for lipids. For instance, the time-varying milk duct dilation signal (when based on the absorption coefficient for lipids) may be divided by a time-varying signal responsive to the absorption coefficients for oxyhemoglobin and by a time-varying signal responsive to the absorption coefficients for deoxyhemoglobin in order to normalize the time-varying signal.

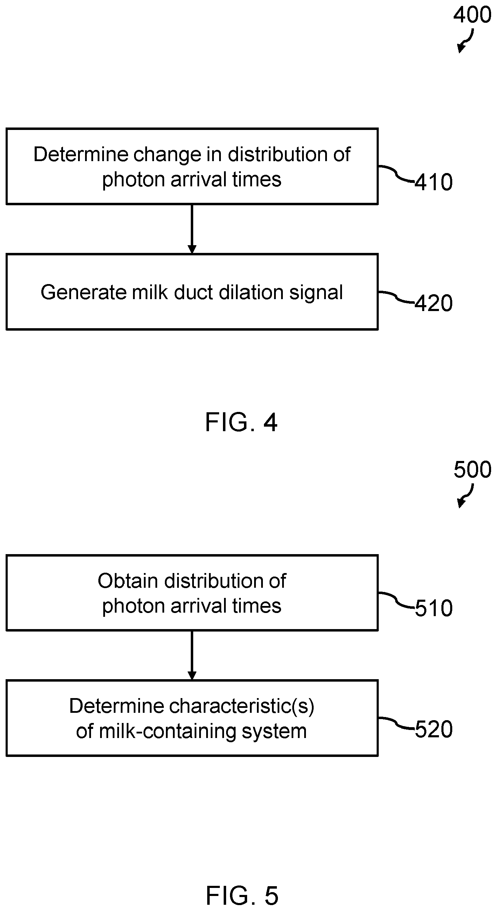

Fig. 4 illustrates a second method 400 for generating a time-varying milk duct dilation signal. This second method for generating a time-varying milk duct dilation is preferred when the SPAD sensor is positioned at a side of the nipple. A minimum of two different wavelengths (i.e. a wavelength within the first wavelength range and a wavelength within the second wavelength range) are required for this second method.

This second method for generating the time-varying milk duct dilation signal is based on the recognition that the distribution of photon arrival times changes as the diameter of the milk duct changes (due to dilation).

The second method 400 begins at step 410, at which the distribution 125 of photon arrival times is processing to determine a change in the distribution over time for each of the first and second wavelength ranges.

Any suitable measure may be used to determine a change in the distribution 125 over time. For instance, the change in the distribution over time may comprise a change in a peak of the distribution or a variation in a time-spread of the distribution. Other suitable measures of the change in the distribution over time will be apparent to the skilled person.

At step 420, the change in the distribution over time for each of the first and second wavelength ranges is processed to generate the time-varying milk duct dilation signal. In other words, the time-varying milk duct dilation signal is a signal responsive to changes in the distribution photon arrival times.

In some examples, the processing system 130 is configured to filter the distribution 125 of photon arrival times prior to generating the time-varying milk duct dilation signal. In other words, the time-varying milk duct dilation signal may, in some examples, be generated based on a filtered distribution 125 of photon arrival times, rather than on the raw distribution.

The distribution 125 of photon arrival times may be filtered based on predetermined anatomical information, to remove the arrival times of photons arriving from depths indicative of breast tissue. The predetermined anatomical information may be determined from population data.

For instance, the predetermined anatomical information may include an expected depth of adipose tissue, and a peak in the distribution of photon arrival times for the wavelength corresponding to lipids absorption at this depth may be used to filter out adipose tissue. In another example, the predetermined anatomical information may include an expected depth of a milk duct, and a peak in the distribution of photon arrival times for the wavelength corresponding to water absorption at this depth may be used to confirm a depth of the milk duct. The distribution may then be filtered to remove photons from other depths.

As stated above, the time-varying milk duct dilation signal may be processed to determine one or more characteristics of the milk-containing system. For instance, a time of a milk ejection reflex event may be determined by detecting a peak in the time-varying milk duct dilation signal. Milk flow information may be determined by detecting changes in the time-varying milk duct dilation signal, which correspond to changes in milk flow.

In some examples, the one or more characteristics may comprise a change in milk supply over time (e.g. a quantification of a long-term natural reduction in milk supply over a period of several months). The processing system 130 may be configured to determine a change in milk supply over time by receiving at least one historical time-varying milk duct dilation signal for the subject, and processing the time-varying milk duct dilation signal and the at least one historical time-varying milk duct dilation signal to determine the change to milk supply over time.

In some examples, the one or more characteristics may comprise a location of a milk duct blockage and/or determine a severity of a milk duct blockage. In order to identify a location of a milk duct blockage and/or determine a severity of a milk duct blockage, a plurality of milk duct dilation signals may be generated, each corresponding to milk duct dilation for a different region.

In some examples, the processing system 130 may be configured to process the distribution 125 of photon arrival times to generate a plurality of milk duct dilation signals, each signal corresponding to a different subset of pixels of the SPAD sensor. The processing system may process the plurality of milk duct dilation signals to determine the one or more characteristics.

In some examples, the at least one SPAD sensor 120 may comprise a plurality of SPAD sensors, each configured to obtain a distribution of photon arrival times for photons reflected from a different region of the breast. For instance, a plurality of SPAD sensors and light sources may be provided in a circular arrangement around the milk-containing system 150. The processing system 130 may be configured to generate a plurality of milk duct dilation signals, each generated using the distribution of photon arrival times of a different SPAD sensor, using any of the methods described above for generating a milk duct dilation signal.

The processing system 130 may spatially correlate the plurality of milk duct dilation signals to identify a location of a milk duct blockage and/or a determine a severity of a milk duct blockage. Suitable methods of spatially correlating the plurality of milk duct dilation signals will be apparent to the skilled person. A lack of correlation in milk duct dilation signals from neighboring regions is indicative of a milk duct blockage. The extent of the lack of correlation is indicative of a severity of a milk duct blockage.

Having determined the one or more characteristics of the milk-containing system using any of the methods described above, the processing system 130 may process the one or more characteristics to generate breastfeeding guidance. The type of breastfeeding guidance generated may depend on the one or more characteristics determined.

For instance, if the one or more characteristics include nutritional information (e.g. a lipids, water and/or beta carotene content of the milk), the breastfeeding guidance may comprise dietary recommendation. If the one or more characteristics include breastfeeding characteristics (e.g. a time of a milk ejection reflex, a milk flow, a location and/or severity of a milk duct blockage, and/or a change in milk supply over time), the breastfeeding guidance may comprise information about milk supply issues and/or milk duct blockages. Further examples of suitable breastfeeding guidance that may be generated based on one or more characteristics of a milk-containing system will be apparent to the skilled person.

In some examples, the generated breastfeeding guidance may be presented to a user (e.g. the mother whose milk is contained in the milk-containing system) by providing a user-perceptible output to an output user interface (not shown in Fig. 1) connected to or forming part of the system 100. For instance, the breastfeeding guidance may be presented to the user in the form of a textual display, an image (e.g. a visualization of a milk duct with an identification of a location of a blockage or a visualization of changes in milk duct diameter over time) and/or a sound. Further examples of suitable user-perceptible outputs will be apparent to the skilled person.

Fig. 5 illustrates a computer-implemented method 500 for determining one or more characteristics of a milk-containing system, according to an embodiment of the invention.

The method 500 begins at step 510, at which a distribution of photon arrival times at a plurality of pixels of at least one SPAD sensor is obtained from the at least one SPAD sensor. The distribution of photon arrival times comprises arrival times of photons emitted by a light source and reflected from within or transmitted through the milk-containing system. The photons comprise first photons having a wavelength within a first wavelength range and second photons having a wavelength within a second, different wavelength range. Each of the first wavelength range and the second wavelength range is a different one of: a wavelength range coinciding with an absorption peak of water, a wavelength range coinciding with an absorption peak of lipids, and a wavelength range coinciding with an absorption peak of beta carotene.

At step 520, the distribution of photon arrival times is processed to determine one or more characteristics of the milk-containing system.

Fig. 6 illustrates a system 600 for determining one or more characteristics of a milk-containing system, according to another embodiment of the invention. The system 600 is identical to the system 100, except that the light source 110 and the at least one SPAD sensor 120 are positioned at a front of the nipple, such that the direction of travel of the photons is substantially parallel to a direction of milk flow. The system 600 is particularly advantageous when using the first method 200 (described with respect to Fig. 2) to generate a time-varying milk duct dilation signal, and in particular for obtaining a distribution of photon arrival times for a wavelength within a wavelength range coinciding with an absorption peak of beta carotene, as wavelengths within this wavelength range do not penetrate very deeply into the tissue.

Fig. 7 illustrates a system 700 for determining one or more characteristics of a milk-containing system, according to another embodiment of the invention. The system 700 is identical to the system 100, except that the light source 110 and the at least one SPAD sensor 120 are arranged in a rectilinear set-up, with the light source and the at least one SPAD sensor are provided on opposite sides of the nipple. With this arrangement, the at least one SPAD sensor detects photons emitted by the light source and transmitted through (and scattered by) the milk-containing system 150.

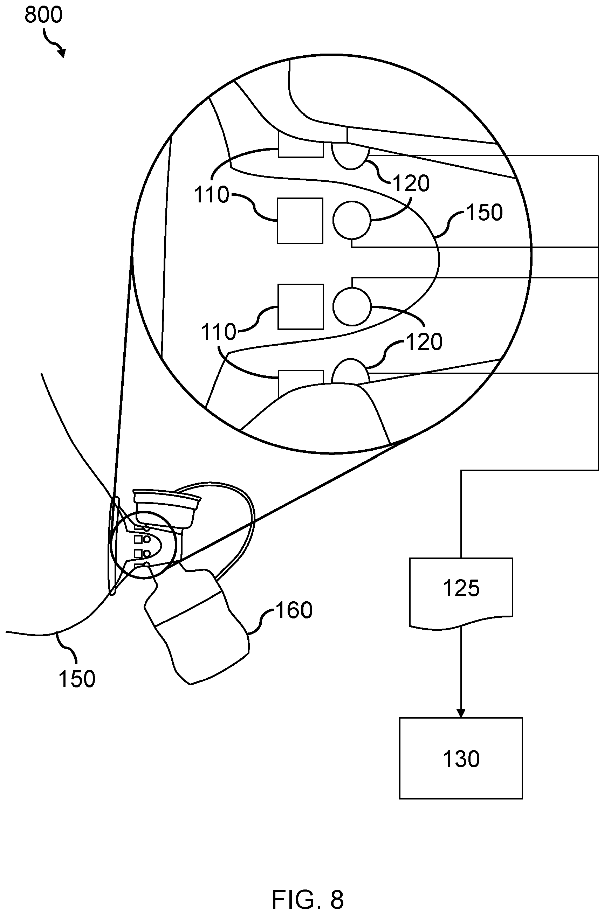

Fig. 8 illustrates a system 800 for determining one or more characteristics of a milk-containing system, according to another embodiment of the invention. The system 800 is identical to the system 100, except that the light source 110 and the at least one SPAD sensor 120 comprise a plurality of light sources 110 and SPAD sensors 120 provided in a circular arrangement around the nipple.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processing system may be implemented by a single processing system or by multiple separate processing units which may together be considered to constitute a "processor". Such processing units may in some cases be remote from each other and communicate with each other in a wired or wireless manner.

Ordinal numbers (e.g. "first", "second" and so on) have been used purely to distinguish different elements from one another for the sake of clarity, and reference to a non-"first" (e.g. "second" or "third") element does not necessitate that a "first" element be present. The skilled person would be capable of relabeling any such elements as appropriate (e.g. relabeling a "second" element as a "first" element if only the second element is present).

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A processing system (130) for determining one or more characteristics of a milk-containing system (150), the processing system being configured to:

   obtain, from at least one SPAD sensor (120), a distribution (125) of photon arrival times at a plurality of pixels of the at least one SPAD sensor for photons emitted by a light source (110) and reflected from within or transmitted through the milk-containing system; and

   process the distribution of photon arrival times to determine one or more characteristics of the milk-containing system,

   wherein the photons comprise first photons having a wavelength within a first wavelength range and second photons having a wavelength within a second, different wavelength range, wherein each of the first wavelength range and the second wavelength range is a different one of the following:

   a wavelength range coinciding with an absorption peak of water;
   a wavelength range coinciding with an absorption peak of lipids; and
   a wavelength range coinciding with an absorption peak of beta carotene.

2. The processing system (130) of claim 1, wherein:

   the milk-containing system (150) is a breast of a human subject; and
   the processing system is configured to determine the one or more characteristics by:

   processing the distribution (125) of photon arrival times to generate a time-varying milk duct dilation signal; and
   processing the time-varying milk duct dilation signal to determine the one or more characteristics.

3. The processing system (130) of claim 2, wherein:
   the photons further comprise third photons having a wavelength within a third wavelength range, different to the first and second wavelength ranges, wherein each of the first wavelength range, the second wavelength range and the third wavelength range is a different one of the following:

   a wavelength range coinciding with an absorption peak of water;
   a wavelength range coinciding with an absorption peak of lipids; and
   a wavelength range coinciding with an absorption peak of beta carotene; and the processing system is configured to generate the milk duct generation signal by:

   for an initial time window:

   processing the distribution (125) of photon arrival times to determine, for each pixel, a first absorption coefficient for each of the first, second and third wavelength ranges;
   processing the first absorption coefficients to determine, for each pixel, a concentration value for each of water, lipids and beta carotene;
   processing the concentration values for each pixel to identify pixels corresponding to a milk duct;

   and, for subsequent time windows:

   processing the data relating to the distribution of photon arrival times at each time window to determine a second absorption coefficient for one of the first, second and third wavelength ranges for at least one pixel of the identified pixels;
   processing each second absorption coefficient for each time window for the at least one pixel to generate the milk duct dilation signal.

4. The processing system (130) of claim 3, wherein:

the photons further comprise fourth photons having a wavelength within a fourth wavelength range coinciding with an absorption peak of oxyhemoglobin and fifth photons having a wavelength within a fifth wavelength range coinciding with an absorption peak of deoxyhemoglobin;

the second absorption coefficient is determined for the wavelength range coinciding with an absorption peak of lipids;

and the processing system is further configured to:

process the distribution (125) of photon arrival times to determine an oxyhemoglobin and deoxyhemoglobin absorption coefficient; and

generate the milk duct dilation signal by processing the second absorption coefficient and the oxyhemoglobin and deoxyhemoglobin absorption coefficient.

5. The processing system (130) of claim 2, wherein the processing system is configured to generate the milk duct dilation signal by:

processing the distribution (125) of photon arrival times to determine a change in the distribution over time for each of the first and second wavelength ranges; and

processing the change in the distribution over time for each of the first and second wavelength ranges to generate the milk duct dilation signal.

6. The processing system (130) of any of claims 2 to 5, wherein the processing system is further configured to filter the distribution (125) of photon arrival times based on predetermined anatomical information.

7. The processing system (130) of any of claims 2 to 6, wherein the processing system is configured to process the time-varying milk duct dilation signal by:

receiving at least one historical time-varying milk duct dilation signal for the subject; and

processing the time-varying milk duct dilation signal and the historical time-varying milk duct signal to determine the one or more characteristics.

8. The processing system (130) of any of claims 2 to 7, wherein the processing system is configured to:

generate a plurality of milk duct dilation signals, each corresponding to a different subset of pixels; and

process the plurality of milk duct dilation signals to determine the one or more characteristics.

9. The processing system (130) of any of claims 2 to 8, wherein:

the at least one SPAD sensor (120) comprises a plurality of SPAD sensors, each configured to obtain a distribution (125) of photon arrival times for photons reflected from a different region of the breast; and

the processing system is configured to:

generate a plurality of milk duct dilation signals, each generated using the distribution of photon arrival times of a different SPAD sensor; and

spatially correlate the plurality of milk duct dilation signals to identify a location of a milk duct blockage.

10. The processing system (130) of any of claims 1 to 9, wherein:

the one or more characteristics comprise a lipids content, a water content and/or a beta carotene content of milk in the milk-containing system;

the photons further comprise third photons having a wavelength within a third wavelength range, different to the first and second wavelength ranges, wherein each of the first wavelength range, the second wavelength range and the third wavelength range is a different one of the following:

a wavelength range coinciding with an absorption peak of water;

a wavelength range coinciding with an absorption peak of lipids; and

a wavelength range coinciding with an absorption peak of beta carotene; and

the processing system is configured to determine the lipids content, water content and/or beta carotene content of milk in the milk-containing system by:

    processing the distribution (125) of photon arrival times to determine an absorption coefficient for each of the first, second and third wavelength ranges;
    processing the absorption coefficients to determine a concentration value for each of water, lipids and beta carotene; and
    processing the concentration values to determine a lipids content, a water content and/or a beta carotene content of milk in the milk-containing system.

11. The processing system (130) of any of claims 1 to 10, wherein the processing system is further configured to process the one or more characteristics to generate breastfeeding guidance.

12. A system (100, 600, 700, 800) for determining one or more characteristics of a milk-containing system (150), the system comprising:

    a light source (110) configured to emit photons towards the milk-containing system, wherein the light source is configured to emit photons comprising first photons having a wavelength within a first wavelength range and second photons having a wavelength within a second, different wavelength range, wherein each of the first wavelength range and the second wavelength range is a different one of the following:

        a wavelength range coinciding with an absorption peak of water;
        a wavelength range coinciding with an absorption peak of lipids; and
        a wavelength range coinciding with an absorption peak of beta carotene;

    at least one SPAD sensor (120), configured to measure an arrival time of photons reflected from within the milk-containing system; and
    the processing system (130) of any of claims 1 to 11.

13. A nipple-interface device (160), comprising:

    a cavity configured to receive a nipple;
    a light source (110) configured to emit photons towards the cavity, wherein the light source is configured to emit photons comprising first photons having a wavelength within a first wavelength range and second photons having a wavelength within a second, different wavelength range, wherein each of the first wavelength range and the second wavelength range is a different one of the following:

        a wavelength range coinciding with an absorption peak of water;
        a wavelength range coinciding with an absorption peak of lipids; and
        a wavelength range coinciding with an absorption peak of beta carotene; and

    at least one SPAD sensor (120), configured to measure an arrival time of reflected photons.

14. A method (500) for determining one or more characteristics of a milk-containing system (150), the method comprising:

    obtaining, from at least one SPAD sensor (120), a distribution (125) of photon arrival times at a plurality of pixels of the at least one SPAD sensor for photons emitted by a light source (110) and reflected from within or transmitted through the milk-containing system, wherein the photons comprise first photons having a wavelength within a first wavelength range and second photons having a wavelength within a second, different wavelength range, wherein each of the first wavelength range and the second wavelength range is a different one of the following:

        a wavelength range coinciding with an absorption peak of water;
        a wavelength range coinciding with an absorption peak of lipids; and
        a wavelength range coinciding with an absorption peak of beta carotene; and

    processing the distribution of photon arrival times to determine one or more characteristics of the milk-containing system.

15. A computer program product comprising computer program code means which, when executed on a computing device having a processing system, cause the processing system to perform all of the steps of the method (500) according to claim 14.

100

150

110

120

150

160

125

130

FIG. 1

200

Determine first absorption coefficients — 210

↓

Determine concentration values — 220

↓

Identify milk duct pixels — 230

↓

Determine second absorption coefficients — 240

↓

Generate milk duct dilation signal — 250

FIG. 2

300

Lipid > 10%?

N ↙        ↘ Y

Beta carotene > 1 μM?        Tissue

N ↙        ↘ Y

Water < 60%?        Tissue

N ↙        ↘ Y

Milk        Tissue

FIG. 3

400

Determine change in distribution of
photon arrival times — 410

Generate milk duct dilation signal — 420

FIG. 4

500

Obtain distribution of
photon arrival times — 510

Determine characteristic(s)
of milk-containing system — 520

FIG. 5

FIG. 6

700

120

110

150

150

160

125

130

FIG. 7

800

110

120

150

110

120

160

150

125

130

FIG. 8

EP 4 439 046 A1

Europäisches
Patentamt

European
Patent Office

Office européen
des brevets

# EUROPEAN SEARCH REPORT

Application Number

EP 23 16 5395

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/087523 A1 (UCL BUSINESS PLC [GB]) 17 May 2018 (2018-05-17) | 1-3,5, 7-10,12, 14,15 | INV. G01N21/31 G01N33/04 |
| Y | * page 4, line 34 - page 19, line 20; figures * | 4,6,11, 13 | |
| Y | RINALDO CUBEDDU ET AL: "Photonics for Life", IEEE PULSE, IEEE, USA, vol. 2, no. 3, 1 May 2011 (2011-05-01), pages 16-23, XP011326601, ISSN: 2154-2287, DOI: 10.1109/MPUL.2011.941519 * the whole document * | 4,6,11, 13 | |
| Y,D | BOSSCHAART NIENKE ET AL: "Diffuse optical spectroscopic imaging of the human lactating breast", PROGRESS IN BIOMEDICAL OPTICS AND IMAGING, SPIE - INTERNATIONAL SOCIETY FOR OPTICAL ENGINEERING, BELLINGHAM, WA, US, vol. 11920, 9 December 2021 (2021-12-09), pages 119200N-119200N, XP060150331, ISSN: 1605-7422, DOI: 10.1117/12.2615233 ISBN: 978-1-5106-0027-0 | 6,11,13 | |
| A | * the whole document * | 1-5, 7-10,12, 14,15 | |

TECHNICAL FIELDS
SEARCHED      (IPC)

G01N

The present search report has been drawn up for all claims

| Place of search | Date of completion of the search | Examiner |
|---|---|---|
| Munich | 18 July 2023 | Politsch, Erich |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 16 5395

This annex lists the patent family members relating to the patent documents cited in the above-mentioned European search report.
The members are as contained in the European Patent Office EDP file on
The European Patent Office is in no way liable for these particulars which are merely given for the purpose of information.

18-07-2023

| Patent document cited in search report | Publication date | Patent family member(s) | Publication date |
|---|---|---|---|
| WO 2018087523 A1 | 17-05-2018 | NONE | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Global Breastfeeding Scorecard, 2019: Increasing commitment to breastfeeding through funding and improved policies and programmes. *WHO/NMH/NHD/19.22*
- **BAN et al.** Kernel Flow: a high channel count scalable time-domain functional near-infrared spectroscopy system. *J Biomed Opt,* 2022, vol. 27 (7), 074710
- **LIEBERT et al.** Evaluation of optical properties of highly scattering media by moments of distributions of times of flight of photons. *Applied Optics,* 2003, vol. 42 (28), 5785-5792

- **NACHABÉ et al.** Diagnosis of breast cancer using diffuse optical spectroscopy from 500 to 1600 nm: comparison of classification methods. *J Biomed Opt,* 2011, vol. 16 (8), 087010
- **BOSSCHAART et al.** Diffuse optical spectroscopic imaging for the investigation of human lactation physiology: a case study on mammary involution. *J Biomed Opt,* 2019, vol. 24 (5), 1-8
- **XAVIER et al.** In Vitro Digestion of Human Milk: Influence of the Lactation Stage on the Micellar Carotenoids Content. *Antioxidants,* 2019, vol. 8 (8), 291